# EUROPÄISCHE PATENTSCHRIFT

(11) **EP 0 650 382 B1**
(45) Veröffentlichungstag und Bekanntmachung des Hinweises auf die Patenterteilung: **23.04.1997**
(21) Anmeldenummer: 93914623.9
(22) Anmeldetag: 14.07.1993
(51) Int. Cl.: A61N 1/375, A61N 1/05, H01R 13/52

(54) **MIKROSTECKVERBINDUNG SOWIE VORRICHTUNG UND VERFAHREN ZUR HERSTELLUNG EINES MIKROSTECKVERBINDUNGSELEMENTES**
MICRO-PLUG CONNECTOR AND DEVICE AND PROCESS FOR PRODUCING A MICRO-PLUG CONNECTOR COMPONENT
MICROCONNECTEUR ET DISPOSITIF ET PROCEDE POUR LA REALISATION D'UN ELEMENT MICROCONNECTEUR

(30) Priorität: 14.07.1992 DE 4223152
(43) Veröffentlichungstag der Anmeldung: 03.05.1995
(73) Patentinhaber: SCHWARZ PHARMA AG, D-40789 Monheim/Rhld. (DE)
(72) Erfinder: JASCH, Ingolf, D-42799 Leichlingen (DE)
(86) Internationale Anmeldenummer: DE9300620
(87) Internationale Veröffentlichungsnummer: WO9401175

(56) Entgegenhaltungen:
- EP-A- 0 329 112
- EP-A- 0 331 959
- FR-A- 2 115 253
- US-A- 4 935 583

## Beschreibung

Die Erfindung betrifft eine Vorrichtung und ein Verfahren zur Herstellung eines Mikrosteckverbindungselements. Eine wenigstens ein erfindungsgemäßes Mikrosteckverbindungselement aufweisende elektrische Versorgungsleitung kann insbesondere für Messungen in der Human- bzw. Veterinärmedizin in bzw. an einem lebenden Körper angebracht werden kann.

Aus der DE-OS 33 31 620 ist eine implantierbare Steckverbindung bekannt, die aus einem Steckerelement und einer Kupplung besteht und zum Anschluß einer implantierbaren medizinischen Einrichtung dient. Diese Einrichtung besitzt ein Behältnis mit einem Gehäuse und einer mit diesem einstückigen Kupplung, die aus Kunststoff formgepreßt ist. Mit der Kupplung sind die Steckerelemente verbunden, an die Elektrodenleitungen angeschlossen sind. Die Kupplung besitzt mehrere elektrische Anschlußteile und eine Aufnahmeöffnung für eine Befestigungsschraube, mit der das Steckerelement an der Kupplung befestigt werden kann.

Der Raumbedarf und das Gewicht der bekannten Steckverbindung ist aufgrund der Vielzahl von Bauelementen sehr hoch. Zum Ein- bzw. Anbringen dieser Steckverbindungen ist daher ein größerer chirurgischer Eingriff notwendig. Darüberhinaus vermittelt die implantierte Steckverbindung beim Träger ein unangenehmes und störendes Tragegefühl. Hinzu kommt, daß die bekannte Steckverbindung im Körper Abwehrreaktionen auslösen kann.

Es ist außerdem bekannt, implantierte medizinische Instrumente, wie beispielsweise Herzkatheter, über eine elektrische Versorgungsleitung, die aus dem Körper herausführt, elektrisch zu versorgen und die gemessenen Signale über diese Versorgungsleitung zu empfangen. Hierfür ist die Versorgungsleitung an ihrem aus dem Körper ragenden Ende mit einem Stecker oder einer Kupplung versehen. Da die bisher verwendeten Stecker oder Kupplungen relativ schwer und groß sind, besteht die permanente Gefahr, daß sich der Patient an dem Stecker oder der Kupplung verhängt und die Hautschnittöffnung, durch die die Versorgungsleitung geführt ist, aufreißt. Da die Versorgungsleitung nicht an der Haut des Patienten, beispielsweise eines Versuchstieres, befestigt wird, kann es darüberhinaus passieren, daß durch eine auf die Versorgungsleitung ausgeübte Zugkraft der Herzkatheter aus seiner Lage herausgezogen wird, so daß keine genauen Messungen mehr möglich sind und eine Verletzungsgefahr besteht. Darüberhinaus entsteht bei fast jeder Bewegung des Patienten eine Relativbewegung zwischen Haut und Versorgungsleitung. Durch die dadurch bedingte ständige Reibung kann die Hautschnittöffnung nicht verheilen.

Aus dem Dokument EP-A-0 329 112 ist ein implantierbarer elektrischer Leiter zur Stromversorung von im Körperinneren implantierten Vorrichtungen bekannt. Dieser Leiter besteht aus einem Draht, der vornehmlich aus einer Legierung aus bestimmten, nach dem Periodensystem der chemischen Elemente definierten Übergangsmetallen, die mit einer Schicht aus inerten Metallen überzogen sind. Schließlich wird dieser Zweikomponenten-Draht mit einer Hülle aus Polyurethan überzogen. Die Auswahl dieser bestimmten Metalle soll den oxidativen Abbau des Polyurethans verhindern. Polyurethan selbst wird nach diesem Dokument wegen seiner physiologischen Wirkung im Körperinneren, beispielsweise wegen seiner geringeren thrombogenen Wirkung und wegen seiner besseren mechanischen Eigenschaften im Vergleich zu Materialien aus Silikonkautschuk verwendet. Die EP-A offenbart jedoch nicht, wie ein Mikrosteckverbindungselement, das mit einer beliebigen Anzahl von Polen versehen werden kann, mit geringstem Gewicht und kleinstem Raumbedarf bei gleichzeitiger hervorragender mechanischer und elektrischer Leistungsfähigkeit erhalten wird.

Schließlich ist noch aus US-A-4,935,583 eine Steckverbindung, die sich durch eine besondere Auswahl bestimmter Materialien auszeichnet, bekannt. Diese ausgewählten Materialien sollen sicherstellen, daß auch unter Temperaturschwankungen eine feste Bindung im Steckverbinder zwischen elektrischem Leiter und Isolator gewährleistet ist. Danach soll diese feste Bindung dadurch hergestellt werden, daß der elektrische Leiter beispielsweise aus Platin und der Isolierkörper (Isolator) aus einer Legierung aus Magnesiumoxid, Silikat und Aluminium bestehen. In die Zwischenräume zwischen elektrischem Leiter und Isolierkörper wird keramisches Material verbracht, um den elektrischen Leiter an den Isolierkörper zu fixieren. Die feste Haftung (Fixierung) wird dadurch gewährleistet, daß alle verwendeten Materialien im wesentlichen den gleichen thermischen Expansionskoeffizient besitzen, und dadurch bei Temperaturänderungen keine Lockerung der elektrischen Leiter vom Isolierkörper durch unterschiedliche thermische Ausdehnung eintritt.

Die US-A offenbart jedoch nicht, daß Steckverbinder, bestehend aus Kupplung und Stecker, in der Geometrie der dichten Kugelpackung erhalten werden, wenn das Material zur Fixierung gleichzeitig auch als Isolierkörper zu dienen bestimmt ist.

Weiterhin ist der Raumbedarf und das Gewicht aufgrund der Vielzahl von Bauelementen sehr hoch.

Der Erfindung liegt die Aufgabe zugrunde, eine Vorrichtung und ein Verfahren zur Herstellung eines biokompatiblen implantierbaren Mikrosteckverbindungselementes mit geringstem Gewicht und kleinsten Abmessungen zu schaffen, wobei eine elektrische Versorgungsleitung wenigstens ein erfindungsgemäßes Mikrosteckverbindungselement aufweisen kann.

Diese Aufgabe wird durch eine Vorrichtung zur Herstellung eines Mikrosteckverbindungselements gelöst, die aus einer zylindrischen Außenhülse, einem koaxial in der Außenhülse angeordneten zylindrischen Zentralelement, mehreren zwischen der Außenhülse und dem Zentralelement angeordneten zylindrischen Abstandshaltern und aus zylindrischen Montageelementen besteht, die jeweils zwischen zwei benachbarten Abstandshaltern angeordnet sind und die benachbarten Abstandshalter, das Zentralelement und die Außenhülse berühren. Die Montageelemente stehen dabei aus der Anordnung aus Außenhülse, Zentralelement und Abstandshalter hervor.

Durch die erfindungsgemäße Vorrichtung zur Herstellung eines Mikrosteckverbindungselements können Mikrostecker mit mehreren Steckerelementen bzw. Mikrokupplungen mit mehreren Kupplungselementen hergestellt werden, die ein sehr geringes Gewicht und geringe Abmessungen haben. Darüberhinaus sind die Kupplungs- bzw. Steckerelemente der hergestellten Mikrosteckverbindungselemente exakt ausrichtbar.

Das Gewicht einer mit der erfindungsgemäßen Vorrichtung hergestellten vierpoligen Mikrosteckverbindung mit Kupplung und Stecker kann weniger als 0,2 g betragen. Die gesamte Steckverbindung aus Kupplung und Stecker ist dabei weniger als 6 mm lang. Ihre Breite beträgt weniger als 2 mm. Dieses geringe Gewicht und die geringen Abmessungen ermöglichen es, die Mikrosteckverbindung problemlos zu implantieren. Der Körper zeigt dabei keine Abwehrreaktionen.

Ein besonders kompakter Aufbau der erfindungsgemäßen Vorrichtung wird dadurch erreicht, daß die Außenhülse, das Zentralelement und die Abstandshalter gleich lang sind, wobei diese Bauelemente für eine sichere Anordnung miteinander vergossen sein können.

Zur besseren Handhabung der erfindungsgemäßen Vorrichtung kann diese noch mit einem Handgriff versehen werden.

Wenn mit der erfindungsgemäßen Vorrichtung Mikrokupplungen mit mehreren Kupplungshülsen hergestellt werden sollen, sind die Montageelemente vorteilhafterweise zylindrische Montagestifte.

Zur Herstellung eines Mikrosteckers aus mehreren Steckerelementen mit Kontaktstiften und Steckerhülsen ist es dagegen zweckmäßig, die Montageelemente als zylindrische Montagebuchsen auszubilden. Gleichzeitig ist es mit den Montagebuchsen jedoch auch möglich, Mikrokupplungen aus mehreren Kupplungshülsen herzustellen.

Bei einem Verfahren zur Herstellung einer Mikrokupplung aus mehreren Kupplungshülsen mit einer der erfindungsgemäßen Vorrichtung werden die Kupplungshülsen auf die zylindrischen Montageelemente aufgesteckt und ausgerichtet. Danach wird der Zwischenraum zwischen den Kupplungshülsen mit Kleber gefüllt und der Kleber anschließend ausgehärtet. Nach der Aushärtung werden die verklebten Kupplungshülsen von den Montageelementen abgezogen.

Wenn mit der Montagebuchsen aufweisenden Vorrichtung Mikrostecker hergestellt werden sollen, die aus mehreren Stekkerelementen mit Kontaktstiften und Steckerhülsen bestehen, werden die Kontaktstifte in die Montagebuchsen eingesteckt und die Steckerelemente ausgerichtet. Das Ausrichten kann dadurch vereinfacht werden, daß man die Kupplungshülsen einer bereits fertigen Kupplung auf die Steckerelemente aufsteckt. Anschließend wird der Zwischenraum zwischen den Steckerhülsen mit Kleber aufgefüllt und der Kleber ausgehärtet. Danach werden die verklebten Steckerelemente von den Montagebuchsen abgezogen.

Der Kleber ist zweckmäßigerweise ein dielektrischer Kleber, vorzugsweise Permapox®.

Der Kontaktabstand der Kupplungshülsen bzw. der Steckerelemente sollte wenigstens 0,05 mm betragen, um ein Übersprechen zu verhindern, bzw. um eine Spannungsfestigkeit bis maximal 65 V Gleichstrom zu erreichen.

In dem Zwischenraum zwischen den Steckerelementen bzw. den Kupplungshülsen kann weiterhin eine Zentralbohrung vorgesehen werden, um eine Zugentlastung aufzunehmen. Die Zentralbohrung hat vorzugsweise zwei Bohrungsabschnitte mit unterschiedlichen Durchmessern, so daß eine Zugentlastungsschnur, die beispielsweise von einem Kevlarfaden gebildet wird, durch den Bohrungsabschnitt mit kleinerem Durchmesser mit einem Ende durchgeführt wird und dieses Ende verknotet und anschließend mit einem Cyanacrylatkleber verklebt wird. Hierdurch schlägt der Knoten bei einer Zugbelastung der Zugentlastungsschnur an der Abstufung der Bohrungsabschnitte an.

Bei einer elektrischen Versorgungsleitung mit wenigstens einem Mikrosteckverbindungselement, sind dessen Kontaktelemente mit elektrischen Leitern verbunden. Damit diese Versorgungsleitung absolut dicht und darüberhinaus auch gewebeverträglich ist, sind die elektrischen Leiter und das Mikrosteckverbindungselement von einem durchgehenden Schlauch aus Polyurethan umgeben.

Etwaige Zwischenräume zwischen dem Schlauch und dem Mikrosteckverbindungselement werden mit Polyurethankleber gefüllt.

Vorteilhafterweise ist an dem Mikrosteckverbindungselement eine Zugentlastungsschnur angebracht.

Wenn an dem Polyurethanschlauch eine Gaze angebracht ist, die mit der Haut eines Patienten verwachsen kann, wird eine Zugbelastung der Versorgungsleitung nicht direkt auf das versorgte Instrument übertragen. Außerdem wird eine Relativbewegung zwischen Versorgungsleitung und Haut verhindert, so daß die Hautschnittöffnung, durch die die Versorgungsleitung geführt ist, verheilen kann. Die Gaze ist zweckmäßigerweise mittels eines Polyurethanklebers mit dem Schlauch verbunden. Eine solche Gaze ist beispielsweise ein Netz aus Mersilene®.

Wenn bei dieser Versorgungsleitung an jedem Ende ein erfindungsgemäßes Mikrosteckverbindungselement angebracht ist, kann diese Versorgungsleitung bei einem Auswechseln des implantierten medizinischen Instrumentes verbleiben. Ein neu implantiertes Instrument muß nur noch an das im Körper verbliebene Mikrosteckverbindungselement angesteckt werden.

Die an dem Instrument angebrachte Versorgungsleitung weist hierfür zweckmäßigerweise ebenfalls eine Versorgungsleitung auf, deren Mikrosteckverbindungselement mit einem Polyurethanschlauch umgeben ist. An der Stelle an der die beiden Polyurethanschläuche der Mikrosteckverbindungselemente aneinanderstoßen, werden sie mit einem Polyurethankleber verklebt. Auf diese Weise erreicht man eine absolut dichte und gewebeverträgliche Mikrosteckverbindung.

Ausführungsbeispiele der Erfindung werden nachstehen anhand von Zeichnungen näher erläutert. Es zeigt:
- Fig. 1: eine Draufsicht auf eine Vorrichtung zur Herstellung einer vierpoligen Mikrosteckverbindung;
- Fig. 2: den Schnitt II-II von Fig. 1;

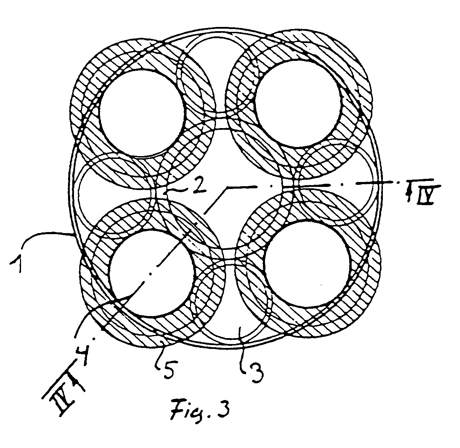

- Fig. 3: die Vorrichtung von Fig. 1 mit darauf angeordneten Kupplungshülsen;
- Fig. 4: den Schnitt IV-IV von Fig. 3;
- Fig. 5: eine Mikrokupplung;
- Fig. 6: eine Draufsicht auf die Mikrokupplung von Fig. 5 und
- Fig. 7: ein in eine Montagebuchse eingestecktes Steckerelement.
- Fig. 8: im Querschnitt ein Ende einer elektrischen Versorgungsleitung mit einer erfindungsgemäßen Mikrokupplung.
- Fig. 9: eine Versorgungsleitung mit einer mit der Haut verwachsbaren Gaze.

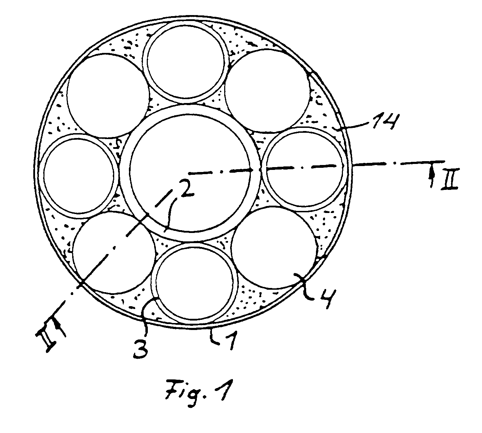

Die in Fig. 1 gezeigte Vorrichtung zur Herstellung einer Mikrosteckverbindung weist eine zylindrische Außenhülse 1 auf, in der koaxial eine Zentralhülse 2 als Zentralelement angeordnet ist. In dem Zwischenraum zwischen der Außenhülse 1 und der Zentralhülse 2 sind abwechselnd vier Montagestifte 4 und vier hülsenförmige Abstandshalter 3 angeordnet. Die Abstandshalter 3 und die Montagestifte 4 haben den gleichen Außendurchmesser, der dem radialen Abstand zwischen der Außenfläche des Zentralelements 2 und der Innenfläche der Außenhülse 1 entspricht. Die Abstandshalter 3 und die Montagestifte 4 berühren demnach jeweils sowohl die Außenfläche der Zentralhülse 2 als auch die Innenfläche der Außenhülse 1. Die Montagestifte 4 sind außerdem so eingeklemmt, daß sie jeweils die zwei benachbarten Abstandshalter 3 berühren.

Zur Sicherung dieser Anordnung ist der Zwischenraum zwischen den einzelnen Bauelementen 1, 2, 3 und 4 der Vorrichtung von Fig. 1 mit einem Kunststoff 14 gefüllt.

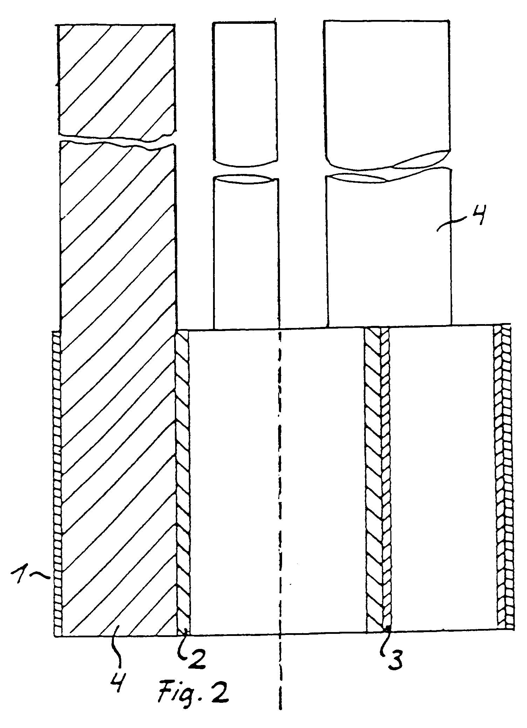

Wie es aus Fig. 2 ersichtlich ist, sind die Außenhülse 1, die Zentralhülse 2 und die Abstandshalter 3 gleich lang. Die Montagestifte 4 sind länger als diese Bauelemente. Die Montagestifte 4 sind so eingesteckt, daß sie an der Unterseite bündig mit der Außenhülse 1, der Zentralhülse 2 und dem Abstandshalter 3 sind. Sie stehen jedoch auf der Oberseite heraus. Die Länge, um die die Montagestifte aus der Anordnung von Zentralhülse 2, Außenhülse 1 und Abstandshalter 3 hervorstehen, entspricht der Länge der Kupplungshülsen 5 einer herzustellenden Mikrokupplung, so daß die Kupplungshülsen 5 über ihre ganze Länge aufgenommen werden.

Zur Herstellung einer Mikrokupplung 30 werden vier Kupplungshülsen 5, beispielsweise die Kupplungshülse TC24S von Miles Roystone Ltd., auf die Montagestifte 4 aufgesteckt, bis sie an der Außenhülse 1 und dem Zentralelement 2 anschlagen. Der Innendurchmesser der Kupplungshülsen 5 entspricht im wesentlichen dem Außendurchmesser der Montagestifte 4.

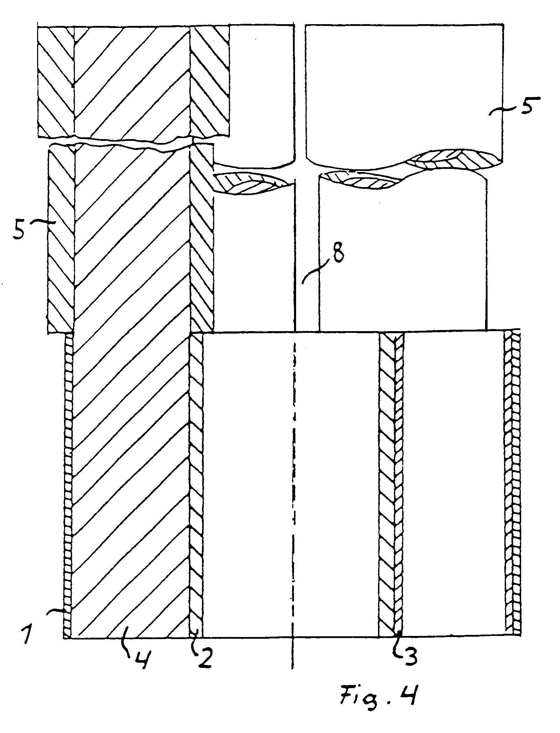

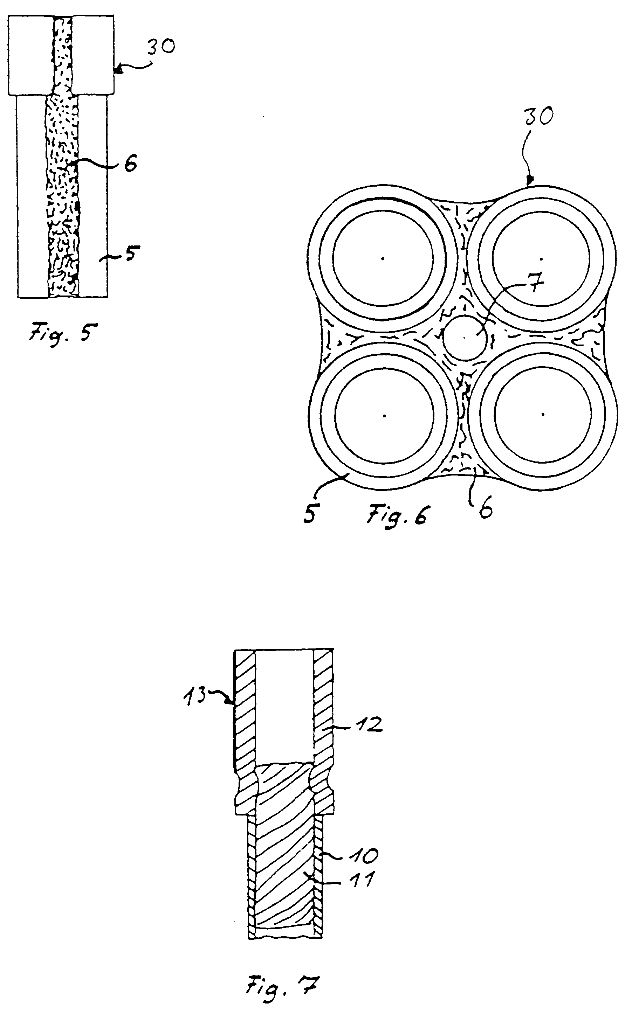

Anschließend werden die Kupplungshülsen 5 nochmals unter einem Stereomikroskop ausgerichtet. Daraufhin wird der Zwischenraum 8 (Fig. 4) zwischen den Kupplungshülsen 5 mittels einer Dosiernadel eines Dosiergerätes mit einem aus Epoxidharz bestehenden dielektrischen Kleber 6, beispielsweise Permapox®, aufgefüllt. Daraufhin wird die Vorrichtung für etwa drei Minuten unter einer Infrarotlampe bei ca. 160 bis 170 Grad und gleichzeitiger Rotation von etwa 60 Umdrehungen pro Minute ausgehärtet. Nach dem Aushärten wird in dem Zwischenraum 8 zwischen den einzelnen Kupplungshülsen eine Zentralbohrung 7 (Fig. 6, Fig. 8) vorgesehen.

Die fertig vergossene Mikrokupplung 30 ist in den Fig. 5 und 6 gezeigt.

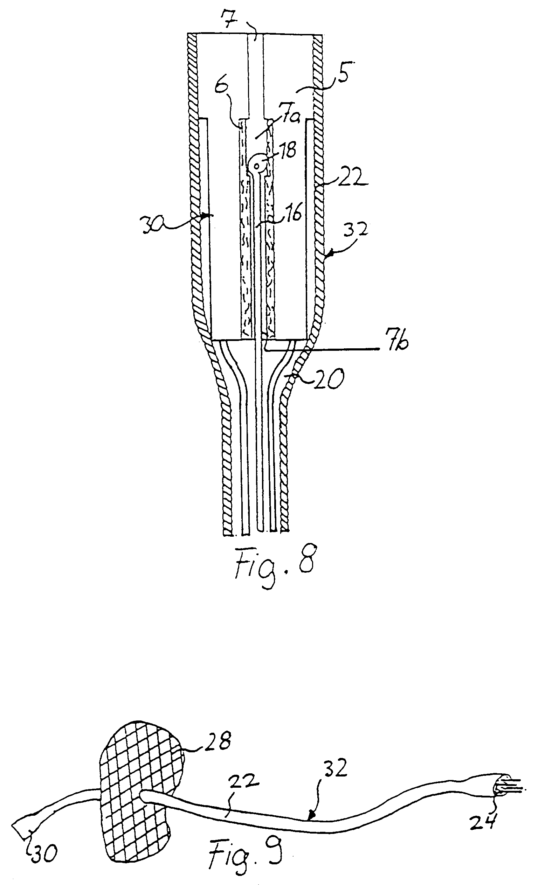

Wie es in Fig. 7 gezeigt ist, können zur Herstellung eines Mikrosteckers anstatt der Montagestifte 4 Montagebuchsen 10 in der Vorrichtung zur Herstellung einer Mikrosteckverbindung angebracht werden. Die Montagebuchsen 10 sind zur Herstellung von Mikrosteckern geeignet, die aus mehreren Steckerelementen bestehen, die eine Steckerhülse 12 und einen darin angebrachten vorstehenden Kontaktstift 11 aufweisen. Ein solches Steckerelement ist beispielsweise das Steckerelement TC24P von Miles Roystone Ltd. Der Außendurchmesser des Kontakstiftes 11 entspricht im wesentlichen dem Innendurchmesser der Montagebuchse 10.

Zur Herstellung des Mikrosteckers werden die Kontaktstifte 11 in die Öffnungen der Montagebuchsen 10 eingesteckt, bis die Steckerhülse 12 an der Montagebuchse anschlägt. Die Verklebung der Steckerhülsen 12 geschieht dann auf die gleiche Weise wie die oben beschriebene Verklebung der Kupplungshülsen 5. Anschließend wird der aus den verklebten Steckerelementen 13 bestehende Mikrostecker aus den Montagebuchsen 10 herausgezogen.

Mit der Montagebuchsen 10 aufweisenden Vorrichtung zur Herstellung einer Mikrosteckverbindung können auch Mikrokupplungen hergestellt werden, deren Kupplungshülsen 5 einen Innendurchmesser aufweisen, der dem Außendurchmesser der Montagebuchsen 10 entspricht.

Die in Fig. 8 gezeigte Versorgungsleitung weist an ihrem einen Ende eine Mikrokupplung 30 auf. Die Kupplungshülsen 5 sind an ihrem Anschlußende jeweils mit gelackten Kupferdrähten 20 verlötet. Für eine bessere Elastizität können die vier Kupferdrähte 20 verdrillt werden, wobei die Lackschicht die Kupferdrähte 20 voneinander isoliert.

Die Zentralbohrung 7 weist zwei Bohrungsabschnitte 7a, 7b auf. Der an das Steckverbindungsende der Mikrokupplung 30 angrenzende Bohrungsabschnitt 7a weist einen größeren Durchmesser auf, wie der anschlußseitige Bohrungsabschnitt 7b.

Vom Anschlußende her ist durch die Zentralbohrung 7 ein Kevlarfaden 16 durchgeführt. Das in dem Bohrungsabschnitt 7a angeordnete Ende des Kevlarfadens 16 ist verknotet und mit Cyanacrylatkleber verklebt. Bei einer Zugbelastung des Kevlarfadens 16 drückt also der Knoten 18 an die Abstufung der Bohrungsabschnitte 7a, 7b.

Die gesamte Anordnung aus Mikrokupplung 30, Kupferdrähten 20 und Kevlarfaden 16 ist von einem Polyurethanschlauch 22 umgeben. Im Bereich der Mikrokupplung 30 ist der Polyurethanschlauch 22 erweitert. Er liegt an der Mikrokupplung 30 an, wobei sein Stirnende bündig mit dem Stirnende der Mikrokupplung 30 ist. Etwaige Zwischenräume zwischen Mikrokupplung 30 und Polyurethanschlauch 22 sind mit Polyurethankleber gefüllt (nicht gezeigt).

Ein Mikrostecker 24 (Fig. 9) kann auf die gleiche Weise an einem Ende der Versorgungsleitung 32 angebracht werden, wie die Mikrokupplung 30.

Die in Fig. 9 gezeigte Versorgungsleitung 32 weist an einem Ende eine Mikrokupplung 30 und an ihrem anderen Ende einen Mikrostecker 24 auf. In der Nähe der Mikrokupplung 30 ist eine netzförmige Gaze 28 befestigt, die mit der Haut eines Patienten verwachsen kann. Die Gaze 28 ist mittels eines Polyurethanklebers mit dem Polyurethanschlauch 22 verbunden.

Zur Anbringung der Versorgungsleitung 32 an dem Patienten wird die Gaze 28 implantiert. Das vibrovasculäre Gewebe wächst dann durch das offene Netz und durchdringt es. Die Versorgungsleitung 32 ist dann fest mit der Haut verbunden.

Mit dem sich in dem Körper des Patienten befindlichen Ende der Versorgungsleitung 32 kann dann eine medizinische Einrichtung, beispielsweise ein Herzkatheder elektrisch verbunden werden, an dem eine weitere Versorgungsleitung angebracht ist, die gleichermaßen ausgebildet ist, wie die Versorgungsleitung 32. Die Stelle, an der die Polyurethanschläuche 22 aneinanderstoßen, wird zur Abdichtung mit Polyurethankleber verklebt.

Die Sicherung der Versorgungsleitung 32 durch die verwachsene Gaze stellt sicher, daß der Herzkatheder bei einer Zugbeanspruchung der Versorgungsleitung 32 außerhalb des Körpers an seiner Stelle verbleibt.

## Patentansprüche

1. Vorrichtung zur Herstellung einer Mikrosteckverbindung, bestehend aus einer zylindrischen Außenhülse (1), einem koaxial in der Außenhülse (1) angeordneten zylindrischen Zentralelement (2), mehreren zwischen der Außenhülse (1) und dem Zentralelement (2) angeordneten zylindrischen Abstandshaltern (3), und zylindrischen Montageelementen (4, 10), die jeweils zwischen zwei benachbarten Abstandshaltern (3) angeordnet sind und die benachbarten Abstandshalter (3), das Zentralelement (2) und die Außenhülse (1) berühren, wobei die Montageelemente (4) aus der Anordnung aus Außenhülse (1), Zentralelement (2) und Abstandshalter (3) vorstehen.

2. Vorrichtung nach Anspruch 1, dadurch gekennzeichnet, daß die Außenhülse (1), das Zentralelement (2) und die Abstandshalter (3) gleich lang sind.

3. Vorrichtung nach Anspruch 1 oder 2, dadurch gekennzeichnet, daß die Außenhülse (1), das Zentralelement (2), die Abstandshalter (3) und die Montageelemente (4, 10) miteinander vergossen sind.

4. Vorrichtung nach Anspruch 3, dadurch gekennzeichnet, daß an der Außenhülse (1) ein Handgriff vorgesehen ist.

5. Vorrichtung nach einem der vorhergehenden Ansprüche, dadurch gekennzeichnet, daß die Montageelemente Montagestifte (4) sind.

6. Vorrichtung nach einem der Ansprüche 1 bis 4, dadurch gekennzeichnet, daß die Montageelemente Montagebuchsen (10) sind.

7. Verfahren zur Herstellung einer Mikrokupplung aus mehreren Kupplungshülsen (5) mit einer Vorrichtung nach einem der vorhergehenden Ansprüche, bei dem die Kupplungshülsen (5) auf die Montageelemente (4, 10) aufgesteckt und ausgerichtet werden, der Zwischenraum zwischen den Kupplungshülsen (5) mit Kleber (6) gefüllt wird, der Kleber (6) ausgehärtet wird und die verklebten Kupplungshülsen (5) von den Montageelementen (4, 10) abgezogen werden.

8. Verfahren zur Herstellung eines Mikrosteckers aus mehreren Kontaktstifte (11) und Steckerhülsen (12) aufweisenden Steckerelementen (13) mit einer Vorrrichtung nach Anspruch 6 bei dem die Kontaktstifte (11) in die Montagebuchsen (10) eingesteckt und die Steckerelemente (13) ausgerichtet werden, der Zwischenraum zwischen den Steckerhülsen (12) mit Kleber (6) gefüllt wird, der Kleber (6) ausgehärtet wird und die verklebten Steckerelemente (13) von den Montagebuchsen (10) abgezogen werden.

9. Verfahren nach Anspruch 7 oder 8, dadurch gekennzeichnet, daß in dem Zwischenraum (8) eine Zentralbohrung (9) zur Aufnahme einer Zugentlastung vorgesehen wird.

10. Mikrosteckverbindung, bestehend aus einem Mikrostecker, der aus Kontaktstiften gebildet ist und einer Mikrokupplung, die aus Kupplungshülsen gebildet ist, dadurch gekennzeichnet, daß jeweils mindestens zwei im Abstand angeordnete Kontaktstifte und Kupplungshülsen mittels eines Klebers, der gleichzeitig der Isolierkörper ist, verklebt sind.

11. Mikrosteckverbindung (24, 30) nach Anspruch 10, dessen Kontaktelemente (5, 13) mit elektrischen Leitern (20) verbunden sind, dadurch gekennzeichnet, daß die elektrischen Leiter (20) und das Mikrosteckverbindungselement (24, 30) von einem durchgehenden Schlauch (22) aus Polyurethan umgeben sind.

12. Mikrosteckverbindung nach Anspruch 11, dadurch gekennzeichnet, daß an der Mikrosteckverbindung eine Zugentlastungsschnur (16) angebracht ist.

13. Mikrosteckverbindung nach Ansprüchen 11 oder 12, dadurch gekennzeichnet, daß an dem Polyurethanschlauch (22) eine Gaze (28) angebracht ist, die mit der Haut eines Patienten verwachsen kann.

## Claims

1. A device for producing a micro plug-in connection, comprising a cylindrical outer sleeve (1), a cylindrical central element (2) arranged coaxially in the outer sleeve (1), a plurality of cylindrical spacers (3) arranged between the outer sleeve (1) and the central element (2), and cylindrical assembly elements (4,10), which are in each case arranged between two neighbouring spacers (3) and are in contact with the neighbouring spacers (3), the central element (2) and the outer sleeve (1), the assembly elements (4) projecting out of the arrangement comprising outer sleeve (1), central element (2) and spacers (3).

2. A device according to claim 1, characterized in that the outer sleeve (1), the central element (2) and the spacers (3) are of equal lengths.

3. A device according to either of claims 1 and 2, characterized in that the outer sleeve (1), the central element (2), the spacers (3) and the assembly elements (4, 10) are cast with one another.

4. A device according to claim 3, characterized in that a handle is provided on the outer sleeve (1).

5. A device according to one of the preceding claims, characterized in that the assembly elements are assembly pins (4).

6. A device according to any of claims 1 to 4, characterized in that the assembly elements are assembly bushes (10).

7. A process for producing a microcoupling comprising a plurality of coupling sleeves (5) by a device according to one of the preceding claims, in which the coupling sleeves (5) are fitted onto the assembly elements (4,10) and aligned, the intermediate space between the coupling sleeves (5) is filled with adhesive (6), the adhesive (6) is cured and the adhesively bonded coupling sleeves (5) are pulled off the assembly elements (4,10).

8. A process for producing a microplug comprising a plurality of plug elements (13) having contact pins (11) and plug sleeves (12) by a device according to claim 6, in which the contact pins (11) are inserted into the assembly bushes (10) and the plug elements (13) are aligned, the intermediate space between the plug sleeves (12) is filled with adhesive (6), the adhesive (6) is cured and the adhesively bonded plug elements (13) are pulled off the assembly bushes (10).

9. A process according to either of claims 7 or 8, characterized in that in the intermediate space (8) there is provided a central bore (9) for receiving a strain release.

10. A micro plug-in connection, comprising a microplug built up by plug elements and a micro coupling built up by coupling sleeves, characterized in that each at least two contact pins and coupling sleeves are arranged in a distance and are adhesively bonded by an adhesive which simultaneously is the insulator.

11. A micro plug-in connection (24,30) according to claim 10, which contact elements (11,13) are connected with electric conductors (20), characterized in that the electric conductors (20) and the micro plug-in connection element (24,30) are surrounded by a continuous tube of polyurethane (22).

12. A micro plug-in connection according to claim 11, characterized in that a strain relief cord (16) is attached to the micro plug-in connection.

13. A micro plug-in connection according to the claims 11 or 12, characterized in that a gauze (28) which can grow together with the skin of a patient is attached to the polyurethane tube (22).

## Revendications

1. Dispositif de fabrication d'un microconnecteur composé d'une gaine externe cylindrique (1), d'un élément central (2) cylindrique dispose coaxialement dans la gaine externe (1) de plusieurs pièces d'écartement (3) cylindriques disposées entre la gaine externe (1) et l'élément central (2) et d'éléments de montage (4, 10) cylindriques qui sont respectivement disposés entre deux pièces d'écartement voisines (3) et touchent respectivement les pièces d'écartement (3) voisines, l'élément central (2) et la gaine externe (1), les éléments de montage (4) débordant du dispositif composé de la gaine externe (1), de l'élément central (2) et des pièces d'écartement (3).

2. Dispositif selon la revendication 1, caractérisé en ce que la gaine externe (1), l'élément central (2) et les pièces d'écartement (3) sont de même longueur.

3. Dispositif selon la revendication 1 ou 2, caractérisé en ce que la gaine externe (1), l'élément central (2), les pièces d'écartement (3) et les éléments de montage (4, 10) sont coulés l'un avec l'autre.

4. Dispositif selon la revendication 3, caractérisé en ce qu'une poignée est prévue sur la gaine externe (1).

5. Dispositif selon l'une des revendications précédentes, caractérisé en ce que les éléments de montage sont des broches de montage (4).

6. Dispositif selon l'une des revendications 1 à 4, caractérisé en ce que les éléments de montage sont des douilles de montage (10).

7. Procédé de fabrication d'un microconnecteur composé de plusieurs manchons d'accouplement (5) avec un dispositif selon l'une des revendications précédentes, les manchons d'accouplement (5) étant enfichés et alignés sur les éléments de montage (4, 10), l'intervalle entre les manchons d'accouplement (5) étant rempli de colle (6), la colle (6) étant durcie et les manchons d'accouplement (5) collés étant extraits des éléments de montage (4, 10).

8. Procédé de fabrication d'un microconnecteur à partir d'éléments connecteurs (13) présentant plusieurs broches de contact (11) et des manchons de connecteur (12) avec un dispositif selon la revendication 6, les broches de contact (11) étant enfichées dans les douilles de montage (10) et les éléments connecteurs (13) étant alignés, l'intervalle entre les manchons du connecteur (12) étant rempli de colle (6), la colle (6) étant durcie et les éléments connecteurs collés (13) étant extraits des douilles de montage (10).

9. Procédé selon la revendication 7 ou 8, caractérisé en ce qu'un alésage central (9) est prévu dans l'intervalle (8) en vue de recevoir un dispositif de décharge de traction.

10. Microconnecteur composé d'un élément microconnecteur qui est formé de broches de contact et d'une microprise, formée de manchons d'accouplement caractérisé en ce qu'au moins deux broches de contact et manchons d'accouplement espacés sont collés à l'aide d'une colle qui est simultanément le corps isolant.

11. Microconnecteur (24, 30) selon la revendication 10 dont les éléments de contact (5, 13) sont reliés à des câbles électriques (20), caractérisé en ce que les câbles électriques (20) et l'élément microconnecteur (24, 30) sont entourés par un flexible continu (22) en polyuréthanne.

12. Microconnecteur selon la revendication 11, caractérisé en ce qu'un câble de décharge de traction (16) est appliqué contre le microconnecteur.

13. Microconnecteur selon les revendications 11 ou 12, caractérisé en qu'une gaze (28) qui peut adhérer à la peau d'un patient est appliquée contre le flexible en polyuréthanne (22).
